# EUROPÄISCHE PATENTSCHRIFT

(11) **EP 1 176 133 B1**
(45) Veröffentlichungstag und Bekanntmachung des Hinweises auf die Patenterteilung: **27.12.2006**
(21) Anmeldenummer: 01116470.4
(22) Anmeldetag: 07.07.2001
(51) Int. Cl.: C07C 51/58, C07C 63/70, C07C 201/12, C07C 205/57, C07C 205/58

(54) **Verfahren zur Herstellung von substituierten Benzoylchloriden**
Process for the preparation of substituted benzoyl chlorides
Procédé pour la préparation de chlorure de benzoyle substitué

(30) Priorität: 26.07.2000 DE 10036653
(43) Veröffentlichungstag der Anmeldung: 30.01.2002
(73) Patentinhaber: Clariant Produkte (Deutschland) GmbH, 65929 Frankfurt am Main (DE)
(72) Erfinder: Bierer, Lars, Dr., 65835 Liederbach (DE); Ritzer, Joachim, Dr., 63517 Rodenbach (DE); Schiemenz, Berthold, Dr., 65929 Frankfurt am Main (DE); Wessel, Thomas, Dr., 61138 Niederdorfelden (DE)

(56) Entgegenhaltungen:
- EP-A- 0 849 253
- EP-A- 0 922 693
- GB-A- 2 061 257
- US-A- 3 274 242

## Beschreibung

Die vorliegende Erfindung betrifft ein Verfahren zur Herstellung von substituierten Benzoylchloriden durch Chlorierung der entsprechenden Benzaldehyde. Substituierte Benzoylchloride lassen sich als aktivierte Derivate der entsprechenden Benzoesäuren betrachten. Aus diesem Grund finden sie als wertvolle Synthesebausteine eine breite Anwendung, z. B. als Zwischenprodukte für die Synthese von Pflanzenschutzmitteln und Arzneistoffen sowie zur Herstellung von Farb- und Kunststoffen.
Aromatische Carbonsäurechloride stellt man üblicherweise durch Umsetzung von Carbonsäuren mit einem Chlorierungsmittel oder durch teilweise Hydrolyse von Benzotrichloriden her. Ein wichtiger Vorteil dieser Verfahren beruht darauf, dass die entsprechenden Ausgangsmaterialien, weil einfach zugänglich, in technischem Maßstab verfügbar sind. Diese günstigen Voraussetzungen sind bei der Herstellung substituierter Benzoylchloride nicht gegeben, da die entsprechenden Ausgangsstoffe, nämlich entsprechend substituierte Carbonsäuren oder Benzotrichloride in der Regel nicht leicht zugänglich sind und auch im Normalfall nicht in technischen Mengen zur Verfügung stehen.
Es ist bekannt, dass man ausgehend von Aldehyden durch Halogenierung der Aldehydgruppe direkt zu Carbonsäurehalogeniden gelangen kann.

So wird in der EP 0 723 950 B1 ein Verfahren zur Herstellung von Alkylbenzoylchloriden durch Chlorierung von Alkylbenzaldehyden beschrieben. Obwohl hierbei in Gegenwart eines Lösungsmittels sowie eines weiteren methyl-oder chlormethylsubstituierten Benzolderivats gearbeitet wird, übersteigt die Selektivität in keinem Fall 86 %. Durch die Verwendung eines komplexen Stoffgemisches (mindestens 4 Komponenten) sowie die nicht sehr hohen Selektivitäten erfordert die Reinigung des Reaktionsproduktes einen hohen Aufwand und die Produktqualität wird ungünstig beeinflusst.
Auch die Reaktion von Alkylbenzaldehyden mit Chlor in Anwesenheit eines Lösungsmittels und eines inerten Gases führt nur zu mäßigen Selektivitäten an Benzoylchloriden (siehe Beispiel 3 in EP 0 849 253 A1: 81 %). Dadurch wird die technische Durchführbarkeit unwirtschaftlich.
Ein Verfahren zur Herstellung von 3-Chlor-4-fluorbenzoylchlorid wird in EP 0 922 693 A1 beschrieben. Hierbei wird die Vorstufe 4-Fluorbenzoylchlorid durch Chlorierung von 4-Fluorbenzaldehyd hergestellt. Es bilden sich jedoch noch 8,3 % einer Nebenkomponente (dort als 4,4'-Difluorbenzil ausgewiesen), die die Reinigung des Produkts erschwert. Bei Durchführung der Chlorierung von 4-Fluorbenzaldehyd in Abwesenheit eines Lösungsmittels bildet sich unter starker Schaumbildung ein gallertartiger Feststoff (siehe im experimentellen Teil Vergleichsbeispiel 3). Ein weiterer Nachteil ist die signifikant erhöhte Bildung eines chlorhaltigen Nebenproduktes (kein 4,4'-Difluorbenzil) und die damit verbundene deutlich verschlechterte Selektivität.
Die Verwendung von in ortho-Stellung durch Halogen substituierten Benzaldehyden, also 2-Halogenbenzaldehyden (worin ein Halogen in ortho-Stellung zur Aldehydgruppe angeordnet ist) ergibt ein zusätzliches Problem. Diese Aldehyde tendieren nämlich zum Teil zur Dehalogenierung (siehe im experimentellen Teil Vergleichsbeispiel 1a), wobei Produkte entstehen, die sich häufig sehr schwer abtrennen lassen und somit die Qualität der Endprodukte, an die bei Einsatz in Synthesen für pharmazeutische Produkte oder Pflanzenschutzmittel sehr hohe Anforderungen gestellt werden, ungünstig beeinflussen.
Darüber hinaus wird bzw. werden bei der Chlorierung häufig ein oder mehrere unerwünschte Nebenprodukte (im experimentellen Teil als Nebenkomponente bezeichnet) gebildet, die vermutlich Folgeprodukte von zwischen dem hergestellten Benzoylchlorid und noch nicht umgesetztem Einsatzprodukt (substituierter Benzaldehyd), möglicherweise unter Einwirkung von Chlor, unkontrolliert ablaufenden Reaktionen darstellen. Insbesondere wird ein chlorhaltiges Nebenprodukt gebildet. Wie die Vergleichsbeispiele 1a, 1b, 2a und 2b zeigen, wird dadurch die Selektivität der Reaktion ungünstig beeinflusst.
Im Hinblick auf die vorstehend geschilderten Nachteile und Probleme besteht der Bedarf, ein Verfahren bereitzustellen, das diese Nachteile und Probleme vermeidet, das sich zudem, ohne großen zusätzlichen Aufwand zu erfordern, auf einfache Weise realisieren lässt und die gewünschten Produkte mit hohem Umsatz und hoher Selektivität in hohen Ausbeuten zugänglich macht.
Diese Aufgabe wird überraschenderweise gelöst durch ein Verfahren zur Herstellung substituierter Benzoylchloride der Formel (1) 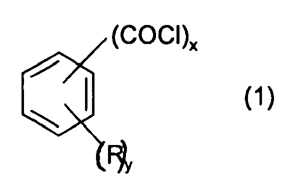 worin R, x und y die nachstehend angegebene Bedeutung haben, dadurch gekennzeichnet, dass man einen Benzaldehyd der Formel (2), worin wenigstens einer der Reste R für ein in ortho-Stellung zu einer Aldehydgruppe angeordnetes Halogen steht, einsetzt 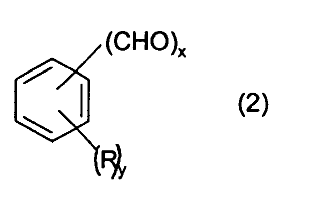 worin R unabhängig voneinander gleich oder verschieden ist und für einen unsubstituierten Phenylrest oder einen durch -Halogen, -NO₂, -CN, -(C₁-C₄)Alkyl oder -(C₁-C₄)Alkoxy substituierten Phenylrest, für -Halogen, -NO₂, -CN, -NR'₂, -OR', -SO₂R', -SO₂OR', -COR' oder -CO₂R' steht, wobei R' ein geradkettiger oder verzweigter C₁-C₁₀-Alkylrest, ein unsubstituierter Phenylrest oder ein durch -Halogen, -NO₂, -CN, -(C₁-C₄)Alkyl oder -(C₁-C₄)Alkoxy substituierter Phenylrest ist, x für 1 oder 2 und y für 1, 2 oder 3 steht, mit einem Chlorierungsmittel in Anwesenheit eines Radikalstarters und eines Lösungsmittels bei 0 bis + 120°C umsetzt, und man als Lösungsmittel Chlorbenzol, 1,2-Dichlorbenzol, 1,3-Dichlorbenzol, 1,4-Dichlorbenzol, 1,2,3-Trichlorbenzol, 1,2,4-Trichlorbenzol, 1,3,5-Trichlorbenzol, 2-Chlortoluol, 3-Chlortoluol, 4-Chlortoluol oder ein Gemisch derselben einsetzt.
R, x und y haben somit in Formel (1) dieselbe Bedeutung wie in Formel (2).

Das erfindungsgemäße Verfahren hat mehrere Vorteile. Es lässt sich zum einen auf eine größere Zahl substituierter Benzaldehyde anwenden und zum anderen ist es überraschenderweise möglich, die Umsetzung auch bei vergleichsweise tiefen Temperaturen mit gutem Erfolg durchzuführen (siehe Beispiele 1 bis 4). Die tiefen Temperaturen ermöglichen eine besonders schonende Umsetzung, die für empfindliche Benzaldehyde, beispielsweise für zur Dehalogenierung neigende halogenierte Benzaldehyde von besonderem Vorteil ist. Es gelingt durch Anwendung tiefer Temperaturen zwar, die Dehalogenierung auch in Abwesenheit eines Lösungsmittels wirkungsvoll herabzusetzen (siehe Vergleichsbeispiele 1b, 2a und 2b), die Bildung unerwünschter als Nebenkomponente ausgewiesener Nebenprodukte lässt sich jedoch nicht in gewünschtem Umfang verringern. Wie ein Vergleich zwischen Vergleichsbeispiel 1b und Beispiel 1 sowie Vergleichsbeispiel 2b und Beispiel 2 zeigt, führt die erfindungsgemäße Arbeitsweise (in Gegenwart eines Lösungsmittels) zu einer überraschend hohen Reduzierung der unerwünschten Nebenprodukte, die sich nur noch in vernachlässigbaren Mengen bilden. Ein analoges Resultat ist auch bei Vergleichsbeispiel 3 und Beispiel 3, wo die Bildung der Nebenkomponente etwa auf ein Zehntel des in Vergleichsbeispiel 3 gebildeten Wertes herabgesetzt werden kann, festzustellen. Auch hier liegt eine drastische Reduzierung der unerwünschten Nebenprodukte, ausgewiesen als Nebenkomponente, vor.
Wie die Beispiele 5, 6 und 7 beweisen, lassen sich in ortho-Stellung zur Aldehydgruppe halogenierte Benzaldehyde auch bei höheren Temperaturen umsetzen, wobei keine Dehalogenierung eintritt und zugleich die Bildung unerwünschter Nebenprodukte nur in geringem bis sehr geringem Ausmaß erfolgt.

Man kann in das erfindungsgemäße Verfahren mit gutem Erfolg einen Benzaldehyd der Formel (2), worin R unabhängig voneinander gleich oder verschieden ist und für -Halogen, -NO₂, -CN, -NR'₂, -OR', -SO₂R', -SO₂OR', -COR' oder -CO₂R' steht, wobei R' ein geradkettiger oder verzweigter C₁-C₄-Alkylrest oder ein unsubstituierter Phenylrest ist, einsetzen. Insbesondere kann man einen Benzaldehyd der Formel (2), worin R unabhängig voneinander gleich oder verschieden ist und für -Halogen, -NO₂, -CN, -OR' oder -COR' steht, wobei R' ein geradkettiger oder verzweigter C₁-C₄-Alkylrest oder ein unsubstituierter Phenylrest ist, einsetzen.
Von besonderem Interesse sind Benzaldehyde (2), worin x für 1 steht.
Von Interesse sind ferner Benzaldehyde (2), worin y für 1 oder 2 oder für 2 oder 3 steht, insbesondere worin y für 1 oder 2, bevorzugt für 2 steht.
Ebenfalls kann man mit gutem Erfolg einen Benzaldehyd (2), worin wenigstens einer der Reste R für ein in ortho-Stellung zu einer Aldehydgruppe angeordnetes Halogen, insbesondere -F, -Cl oder -Br, bevorzugt -F oder -Cl ist, steht, insbesondere worin einer der Reste R für ein in ortho-Stellung zu einer Aldehydgruppe angeordnetes Halogen und ein zweiter der Reste R ebenfalls für Halogen steht und Halogen -F, -Cl oder -Br, insbesondere -F oder -Cl ist, einsetzen.
Gut geeigneter Benzaldehyd (2) ist, worin R unabhängig voneinander gleich oder verschieden ist und für -F, -Cl oder -NO₂ steht, x für 1 und wenigstens einer der Reste R für ein in ortho-Stellung zu einer Aldehydgruppe angeordnetes -F oder -Cl steht.
Man setzt üblicherweise den substituierten Benzaldehyd (2) und das Chlorierungsmittel im Molverhältnis 1 : (0,5 bis 2,0), insbesondere 1 : (0,7 bis 1,5), bevorzugt 1 : (0,9 bis 1,2) ein.
Als Chlorierungsmittel kann man Chlor, oder ein chlorabgebendes Agens, gegebenenfalls in Gegenwart eines inerten Verdünnungsgases wie Stickstoff, Kohlendioxid oder eines Edelgases, einsetzen. Vorzugsweise wird in Abwesenheit eines inerten Verdünnungsgases gearbeitet. Geeignet als Chlorierungsmittel, ohne Anspruch auf Vollständigkeit zu erheben, sind Cl₂, SOCl₂, SO₂Cl₂, PCl₃, POCl₃, PCl₅, SbCl₅, ICl, ICl₃, SCl₂, S₂Cl₂, MnCl₄, (C₁-C₄)Alkylhypochlorit, CCl₄ und N-Chlorsuccinimid oder ein Gemisch derselben.
Man setzt insbesondere Cl₂, SOCl₂, SO₂Cl₂, oder ein Gemisch derselben als Chlorierungsmittel ein.
Es hat sich als besonders günstig erwiesen, den substituierten Benzaldehyd (2) mit Cl₂ als Chlorierungsmittel umzusetzen.
Die Umsetzung von substituierten Benzaldehyden mit dem Chlorierungsmittel erfolgt nach Art einer radikalischen Chlorierung in Anwesenheit eines Radikalstarters. Üblicherweise verwendet man ein Peroxid oder eine Azoverbindung einzeln oder in Kombination miteinander als Radikalstarter. Es ist bekannt, dass organische Peroxide und organische Azoverbindungen unter dem Einfluss von Wärme und/oder Licht in Radikale zerfallen, die die radikalische Chlorierung initiieren.
Beispiele für geeignete Peroxide und organische Azoverbindungen sind, ohne Anspruch auf Vollständigkeit zu erheben, Ethylmethylketonperoxid, tert.-Butylhydroperoxid, tert.-Butyl-trimethylsilylperoxid, Cumolhydroperoxid, Lauroylperoxid, Dibenzoylperoxid, Di-tert.-Butylperoxid, Dilaurylperoxid, Perbenzoesäure-tert.-butylester, tert.-Butylperoxy-2-ethylhexanoat, 2,2'-Azobis(4-methoxy-2,4-dimethylvaleronitril), 2,2'-Azobis(2,4-dimethylvaleronitril), 2,2'-Azobis(isobutyronitril), Dimethyl-2,2'-azobis(isobutyrat), 2,2'-Azobis(2-methylbutyronitril), 1,1'-Azobis(1-cyclohexancarbonitril), 2-(Carbamoylazo)-isobutyronitril, 2,2'-Azobis(2,4,4-trimethylpentan), 2,2'-Azobis(isobutyroamidin)-Dihydrochlorid, 2,2'-Azobis(N,N'-dimethylenisobutyramidin)-Dihydrochlorid, 2,2'-Azobis(2-amidinopropan)-Dihydrochlorid, 2,2'-Azobis(N,N'-dimethylenisobutyramidin), 4,4'-Azobis(4-cyanpentansäure) und/oder 2,2'-Azobis[2-methyl-N-(2-hydroxyethyl)propionsäureamid], insbesondere tert.-Butylhydroperoxid, Dibenzoylperoxid, Dilaurylperoxid, tert.-Butylperoxy-2-ethylhexanoat, 2,2'-Azobis(4-methoxy-2,4-dimethylvaleronitril), 2,2'-Azobis(2,4-dimethylvaleronitril) und/oder 2,2'-Azobis(isobutyronitril).
Nach einer bevorzugten Variante setzt man 2,2'-Azobis(2,4-dimethylvaleronitril), 2,2'-Azobis(isobutyronitril) und/oder 2,2'-Azobis(4-methoxy-2,4-dimethylvaleronitril) als Radikalstarter ein.
Man setzt den Radikalstarter (Peroxid und/oder Azoverbindung) üblicherweise in einer Menge von 0,001 bis 10, insbesondere 0,005 bis 5, bevorzugt 0,02 bis 2 Molprozent, bezogen auf den substituierten Benzaldehyd (2) ein.
Die Umsetzung der substituierten Benzaldehyde (2) mit dem Chlorierungsmittel erfolgt in Anwesenheit eines Lösungsmittels.

Man setzt als Lösungsmittel Chlorbenzol, 1,2-Dichlorbenzol, 1,3-Dichlorbenzol, 1,4-Dichlorbenzol, 1,2,3-Trichlorbenzol, 1,2,4-Trichlorbenzol, 1,3,5-Trichlorbenzol, 2-Chlortoluol, 3-Chlortoluol, 4-Chlortoluol oder ein Gemisch derselben, bevorzugt Chlorbenzol, 1,2-Dichlorbenzol, 1,3-Dichlorbenzol, 1,4-Dichlorbenzol oder ein Gemisch derselben ein.
Nach einer besonders bevorzugten Variante verwendet man Chlorbenzol als Lösungsmittel.
Man setzt das Lösungsmittel üblicherweise im Verhältnis 50:1 bis 1:50, insbesondere 10:1 bis 1:10, bevorzugt 4:1 bis 1:4 bezogen auf den substituierten Benzaldehyd ein.
In einer Vielzahl von Fällen hat es sich als ausreichend erwiesen, die substituierten Benzaldehyde (2) mit dem Chlorierungsmittel bei 0 bis 120°C, bevorzugt 20 bis 90°C, besonders bevorzugt 30 bis 85 °C umzusetzen. Eine Vielzahl substituierter Benzaldehyde lässt sich bei einer Temperatur von 35 bis 80°C mit gutem Erfolg umsetzen.
Die Isolierung der erhaltenen substituierten Benzoylchloride (1) kann nach üblichen Aufarbeitungsverfahren erfolgen, die von den Schmelz- und Siedepunkten der Produkte bzw. bei Arbeiten unter Verwendung von Lösungsmitteln von der Löslichkeit des Produktes im verwendeten Lösungsmittel und wiederum dessen Festpunkt/Siedepunkt abhängen. Übliche Aufarbeitungsverfahren sind hierbei Chromatographie, Filtration, Phasentrennung, Zentrifugation oder Destillation bei Normaldruck oder im Vakuum. Insbesondere die destillative Aufarbeitung stellt für substituierte Benzoylchloride die Methode der Wahl dar.
Das erfindungsgemäße Verfahren lässt sich diskontinuierlich oder kontinuierlich durchführen. Alle Verfahrensschritte können unter Normaldruck, Unter- oder Überdruck durchgeführt werden.
Die folgenden Beispiele erläutern das Verfahren, ohne es zu beschränken:

### Experimenteller Teil

### Vergleichsbeispiel 1a:

In einer Säulenchlorierapparatur (Höhe 60 cm, Durchmesser 5 cm) werden unter Schutzgas 552,3 g 2,6-Difluorbenzaldehyd vorgelegt und mit 2,3 g 2,2'-Azobis(2,4-dimethylvaleronitril) unter Rühren versetzt. Anschließend wird auf 67°C Innentemperatur geheizt und es werden innerhalb von 7 Stunden insgesamt 290 g Chlor eingeleitet. Das Chlor wird in einer Menge von 14 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 2,4 % | (a/a) | 2,6-Difluorbenzaldehyd |
| | 85,1 % | (a/a) | 2,6-Difluorbenzoylchlorid |
| | 1,1 % | (a/a) | 2-Fluorbenzoylchlorid |
| | 10,7 % | (a/a) | Nebenkomponente |
| Umsatz: | 97,6 % | | |
| Selektivität: | 87,2 % | | |

### Vergleichsbeispiel 1b:

In einer Säulenchlorierapparatur (Höhe 60 cm, Durchmesser 5 cm) werden unter Schutzgas 552,7 g 2,6-Difluorbenzaldehyd vorgelegt und mit 2,3 g 2,2'-Azobis(2,4-dimethylvaleronitril) unter Rühren versetzt. Anschließend wird auf 51°C Innentemperatur geheizt und es werden innerhalb von 7 Stunden insgesamt 292 g Chlor eingeleitet. Das Chlor wird in einer Menge von 14 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 1,8 % | (a/a) | 2,6-Difluorbenzaldehyd |
| | 92,1 % | (a/a) | 2,6-Difluorbenzoylchlorid |
| | 5,7 % | (a/a) | Nebenkomponente |
| Umsatz: | 98,2 % | | |
| Selektivität: | 93,8 % | | |

### Beispiel 1:

In einer Säulenchlorierapparatur (Höhe 60 cm, Durchmesser 5 cm) werden unter Schutzgas 555,2 g 2,6-Difluorbenzaldehyd in 370 g Chlorbenzol vorgelegt und mit 2,4 g 2,2'-Azobis(2,4-dimethylvaleronitril) unter Rühren versetzt. Anschließend wird auf 51°C Innentemperatur geheizt und es werden innerhalb von 7 Stunden insgesamt 295 g Chlor eingeleitet. Das Chlor wird in einer Menge von 14 Liter/Stunde zugesetzt. Anschließend wird nicht umgesetztes Chlor mit Schutzgas ausgeblasen und das Reaktionsgemisch auf Raumtemperatur abgekühlt. Das Fortschreiten der Umsetzung wird mittels gaschromatographischer Analyse (GC) kontrolliert.

| | | | |
|---|---|---|---|
| GC-Umsatzkontrolle: | 0,2 % | (a/a) | 2,6-Difluorbenzaldehyd |
| | 98,9 % | (a/a) | 2,6-Difluorbenzoylchlorid |
| | 0,8 % | (a/a) | Nebenkomponente |
| Umsatz: | 99,8 % | | |
| Selektivität: | 99,1 % | | |
| Ausbeute: | 96,7 % | | |

Anschließend erfolgt die Produktdestillation im Vakuum über eine Füllkörperkolonne (Höhe 100 cm, Füllung Sulzer CY) mit einem Rücklaufverhältnis von 1:7, wobei 2,6-Difluorbenzoylchlorid bei 145 mbar bei konstant 125°C siedet. Das fraktionierte 2,6-Difluorbenzoylchlorid weist eine Reinheit von > 99,5 % (a/a) auf.

### Vergleichsbeispiel 2a:

In einem 500 ml-Kolben werden unter Schutzgas 249,8 g 2-Fluorbenzaldehyd vorgelegt und mit 0,9 g 2,2'-Azobis(isobutyronitril) unter Rühren versetzt. Anschließend wird auf 59°C Innentemperatur geheizt und es werden innerhalb von 8 Stunden insgesamt 152 g Chlor eingeleitet. Das Chlor wird in einer Menge von 7 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 1,5% | (a/a) | 2-Fluorbenzaldehyd |
| | 65,6 % | (a/a) | 2-Fluorbenzoylchlorid |
| | 31,0 % | (a/a) | Nebenkomponente |
| Umsatz: | 98,5 % | | |
| Selektivität: | 66,6 % | | |

### Vergleichsbeispiel 2b:

In einem 500 ml-Kolben werden unter Schutzgas 247,3 g 2-Fluorbenzaldehyd vorgelegt und mit 1,0 g 2,2'-Azobis(2,4-dimethylvaleronitril) unter Rühren versetzt. Anschließend wird auf 40°C Innentemperatur geheizt und es werden innerhalb von 8 Stunden insgesamt 150 g Chlor eingeleitet. Das Chlor wird in einer Menge von 7 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 1,7 % | (a/a) | 2-Fluorbenzaldehyd |
| | 85,6 % | (a/a) | 2-Fluorbenzoylchlorid |
| | 12,6 % | (a/a) | Nebenkomponente |
| Umsatz: | 98,3 % | | |
| Selektivität: | 87,1 % | | |

### Beispiel 2:

In einem 2 I-Kolben werden unter Schutzgas 248,0 g 2-Fluorbenzaldehyd in 900 g Chlorbenzol vorgelegt und mit 1,0 g 2,2'-Azobis(2,4-dimethylvaleronitril) unter Rühren versetzt. Anschließend wird auf 40°C Innentemperatur geheizt und es werden innerhalb von 8 Stunden insgesamt 150 g Chlor eingeleitet. Das Chlor wird in einer Menge von 7 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 1,0 % | (a/a) | 2-Fluorbenzaldehyd |
| | 98,4 % | (a/a) | 2-Fluorbenzoylchlorid |
| | 0,6 % | (a/a) | Nebenkomponente |
| Umsatz: | 99,0 % | | |
| Selektivität: | 99,4 % | | |

### Vergleichsbeispiel 3:

In einer Säulenchlorierapparatur (Höhe 60 cm, Durchmesser 5 cm; Füllhöhe 55 cm) werden unter Schutzgas 829,1 g 4-Fluorbenzaldehyd vorgelegt und mit 2,7 g (0,16 Molprozent) 2,2'-Azobis(2,4-dimethylvaleronitril) unter Rühren versetzt. Anschließend wird auf 42°C Innentemperatur geheizt und Chlor eingeleitet. Das Chlor wird in einer Menge von 12 Liter/Stunde zugesetzt. Bereits nach 2,5 Stunden bildet sich unter heftigem Schäumen ein gallertartiger Feststoff, der dazu führt, dass die Chloreinleitung nach 13 Stunden abgebrochen werden muss.
Das Fortschreiten der Umsetzung wird mittels gaschromatographischer Analyse (GC) kontrolliert.

| | | | |
|---|---|---|---|
| GC-Umsatzkontrolle: | 10,7 % | (a/a) | 4-Fluorbenzaldehyd |
| | 66,2 % | (a/a) | 4-Fluorbenzoylchlorid |
| | 22,2 % | (a/a) | Nebenkomponente |
| Umsatz: | 89,3 % | | |
| Selektivität: | 74,1 % | | |

### Beispiel 3:

In einer Säulenchlorierapparatur (Höhe 60 cm, Durchmesser 5 cm; Füllhöhe 55 cm) werden unter Schutzgas 402,2 g 4-Fluorbenzaldehyd in 400 g Chlorbenzol vorgelegt und mit 1,3 g (0,16 Molprozent) 2,2'-Azobis(2,4-dimethylvaleronitril) unter Rühren versetzt. Anschließend wird auf 42°C Innentemperatur geheizt und es werden innerhalb von 7 Stunden insgesamt 240 g Chlor eingeleitet. Das Chlor wird in einer Menge von 11 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 0,5 % | (a/a) | 4-Fluorbenzaldehyd |
| | 97,1 % | (a/a) | 4-Fluorbenzoylchlorid |
| | 2,4 % | (a/a) | Nebenkomponente |
| Umsatz: | 99,5 % | | |
| Selektivität: | 97,6 % | | |
| Ausbeute: | 95,3 % | | |

Anschließend erfolgt die Produktdestillation i. Vak. über Füllkörperkolonne (Höhe 100 cm, Füllung Sulzer CY) mit einem Rücklaufverhältnis von 1 : 15, wobei 4-Fluorbenzoylchlorid bei 40 mbar bei konstant 94°C siedet. Das fraktionierte 4-Fluorbenzoylchlorid weist eine Reinheit von > 99,5 % (a/a) auf.

### Beispiel 4:

In einem 1 I-Kolben werden unter Schutzgas 310 g 2-Chlorbenzaldehyd in 300 g Chlorbenzol vorgelegt und mit 1,1 g 2,2'-Azobis(2,4-dimethylvaleronitril) unter Rühren versetzt. Anschließend wird auf 41°C Innentemperatur geheizt und es werden innerhalb von 7 Stunden insgesamt 164 g Chlor eingeleitet. Das Chlor wird in einer Menge von 8 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 5,0 % | (a/a) | 2-Chlorbenzaldehyd |
| | 93,0 % | (a/a) | 2-Chlorbenzoylchlorid |
| | 1,1 % | (a/a) | Nebenkomponente |
| Umsatz: | 95,0 % | | |
| Selektivität: | 97,9 % | | |

### Beispiel 5:

In einer Säulenchlorierapparatur (Höhe 60 cm, Durchmesser 5 cm) werden unter Schutzgas 200,4 g 2,6-Dichlorbenzaldehyd in 650 g Chlorbenzol vorgelegt und mit 1,2 g 2,2'-Azobis(isobutyronitril) unter Rühren versetzt. Anschließend wird auf 80°C Innentemperatur geheizt und es werden innerhalb von 6 Stunden insgesamt 85 g Chlor eingeleitet. Das Chlor wird in einer Menge von 5 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 0,1% | (a/a) | 2,6-Dichlorbenzaldehyd |
| | 99,0 % | (a/a) | 2,6-Dichlorbenzoylchlorid |
| | 0,9% | (a/a) | Nebenkomponente |
| Umsatz: | 99,9 % | | |
| Selektivität: | 99,1 % | | |

### Beispiel 6:

In einer Säulenchlorierapparatur (Höhe 60 cm, Durchmesser 5 cm) werden unter Schutzgas 200,6 g 2-Chlor-6-fluorbenzaldehyd in 600 g Chlorbenzol vorgelegt und mit 1,0 g 2,2'-Azobis(isobutyronitril) unter Rühren versetzt. Anschließend wird auf 70°C Innentemperatur geheizt und es werden innerhalb von 7 Stunden insgesamt 95 g Chlor eingeleitet. Das Chlor wird in einer Menge von 5 Liter/Stunde zugesetzt. Anschließend wird nicht umgesetztes Chlor mit Schutzgas ausgeblasen und das Reaktionsgemisch auf Raumtemperatur abgekühlt.
Das Fortschreiten der Umsetzung wird mittels gaschromatographischer Analyse (GC) kontrolliert.

| | | | |
|---|---|---|---|
| GC-Umsatzkontrolle: | 0,2 % | (a/a) | 2-Chlor-6-fluorbenzaldehyd |
| | 98,4% | (a/a) | 2-Chlor-6-fluorbenzoylchlorid |
| | 1,2 % | (a/a) | Nebenkomponente |
| Umsatz: | 99,8 % | | |
| Selektivität: | 98,6 % | | |

### Beispiel 7:

In einem 500ml-Kolben werden unter Schutzgas 50,1 g 2-Chlor-5-nitrobenzaldehyd in 200 g Chlorbenzol vorgelegt und mit 0,6 g 2,2'-Azobis(isobutyronitril) unter Rühren versetzt. Anschließend wird auf 80°C Innentemperatur geheizt und es werden innerhalb von 3 Stunden insgesamt 20 g Chlor eingeleitet. Das Chlor wird in einer Menge von 3 Liter/Stunde zugesetzt. Anschließend wird nicht umgesetztes Chlor mit Schutzgas ausgeblasen und das Reaktionsgemisch auf Raumtemperatur abgekühlt. Das Fortschreiten der Umsetzung wird mittels gaschromatographischer Analyse (GC) kontrolliert.

| | | | |
|---|---|---|---|
| GC-Umsatzkontrolle: | 55,1 % | (a/a) | 2-Chlor-5-nitrobenzaldehyd |
| | 42,3 % | (a/a) | 2-Chlor-5-nitrobenzoylchlorid |
| | 1,6 % | (a/a) | Nebenkomponente |
| Umsatz: | 44,9 % | | |
| Selektivität: | 94,2 % | | |

## Patentansprüche

1. Verfahren zur Herstellung substituierter Benzoylchloride der Formel (1) 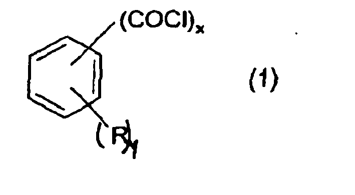 worin R, x und y die nachstehend angegebene Bedeutung haben, **dadurch gekennzeichnet, dass** man einen Benzaldehyd der Formel (2), worin wenigstens einer der Reste R für ein in ortho-Stellung zu einer Aldehydgruppe angeordnetes Halogen steht, einsetzt. 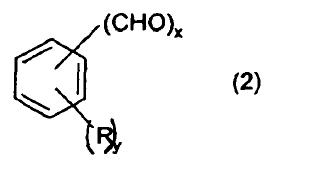 worin R unabhängig voneinander gleich oder verschieden ist und für einen unsubstituierten Phenylrest oder einen durch -Halogen, -NO₂, -CN, -(C₁-C₄)Alkyl oder -(C₁-C₄)Alkoxy substituierten Phenylrest, für -Halogen, -NO₂, -CN, -NR'₂, -OR', -SO₂R', -SO₂OR', -COR' oder -CO₂R' steht, wobei R' ein geradkettiger oder verzweigter C₁-C₁₀-Alkylrest, ein unsubstituierter Phenylrest oder ein durch -Halogen, -NO₂, -CN, -(C₁-C₄)Alkyl oder -(C₁-C₄)Alkoxy substituierter Phenylrest ist, x für 1 oder 2 und y für 1, 2 oder 3 steht, mit einem Chlorierungsmittel in Anwesenheit eines Radikalstarters und eines Lösungsmittels bei 0-120 °C umsetzt, und man als Lösungsmittel Chlorbenzol, 1,2-Dichlorbenzol, 1,3-Dichlorbenzol, 1,4-Dichlorbenzol, 1,2,3-Trichlorbenzol, 1,2,4-Trichlorbenzol, 1,3,5-Trichlorbenzol, 2-Chlortoluol, 3-Chlortoluol oder ein Gemisch derselben einsetzt.

2. Verfahren nach Anspruch 1, **dadurch gekennzeichnet, dass** man einen Benzaldehyd der Formel (2), worin R unabhängig voneinander gleich oder verschieden ist und für -Halogen, -NO₂, -CN, -NR'₂, -OR', -SO₂R', -SO₂OR', -COR' oder -CO₂R' steht, wobei R' ein geradkettiger oder verzweigter C₁-C₄-Alkylrest oder ein unsubstituierter Phenylrest ist, einsetzt.

3. Verfahren nach Anspruch 1, **dadurch gekennzeichnet, dass** man einen Benzaldehyd (2), worin x für 1 steht, einsetzt.

4. Verfahren nach einem oder mehreren der Ansprüche 1 bis 3 **dadurch gekennzeichnet, dass** man einen Benzaldehyd (2), worin R für-F -Cl oder -NO₂ steht, x für 1 und wenigstens einer der Reste R für ein in ortho-Stellung zu der Aldehydgruppe angeordnetes -F oder -Cl steht, einsetzt.

5. Verfahren nach einem oder mehreren der Ansprüche 1 bis 4, **dadurch gekennzeichnet, dass** man als Chlorierungsmittel Cl₂, SOCl₂. SO₂Cl₂, PCl₃, POCl₃, PCl₅, SbCl₅, ICl, ICl₃, SCl₂, S₂Cl₂, MnCl₄, (C₁-C₄)Alkylhypochlorit, CCl₄, N-Chlorsuccinimid oder ein Gemisch derselben einsetzt.

6. Verfahren nach einem oder mehreren der Ansprüche 1 bis 5, **dadurch gekennzeichnet, dass** man als Chlorierungsmittel Cl₂, SOCl₂, SO₂Cl₂ oder ein Gemisch derselben einsetzt.

7. Verfahren nach einem oder mehreren der Ansprüche 1 bis 6, **dadurch gekennzeichnet, dass** man als Radikalstarter ein organisches Peroxid oder eine organische Azoverbindung oder ein Gemisch derselben einsetzt.

8. Verfahren nach einem oder mehreren der Ansprüche 1 bis 7, **dadurch gekennzeichnet, dass** man 2,2'-Azobis(2,4-dimethylvaleronitril), 2,2'-Azobis (isobutyronitril), 2,2'-Azobis(4-methoxy-2,4-dimethylvaleronitril) oder ein Gemisch derselben als Radikalstarter einsetzt.

9. Verfahren nach einem oder mehreren der Ansprüche 1 bis 8, **dadurch gekennzeichnet, dass** man die Umsetzung bei 20 bis 90°C durchführt.

## Claims

1. A process for preparing substituted benzoyl chlorides of the formula (1) 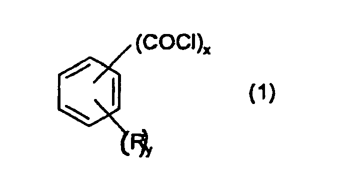 where R, x and y have the meaning given below, which comprises reacting a benzaldehyde of the formula (2), where at least one of the radicals R is a halogen in an ortho position to an aldehyde group, 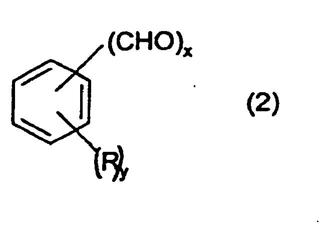 where R, independently of each other, is identical or different and is an unsubstituted phenyl radical or a phenyl radical that is substituted by halogen, NO₂, CN, (C₁-C₄) alkyl or (C₁-C₄) alkoxy, or is halogen, NO₂, CN, NR'₂, OR', SO₂R', SO₂OR', COR' or CO₂R', where R' is an unbranched or branched C₁-C₁₀ alkyl radical, an unsubstituted phenyl radical or a phenyl radical which is substituted by halogen, NO₂, CN, (C₁-C₄)alkyl or (C₁-C₄) alkoxy, x is 1 or 2 and y is 1, 2 or 3, with a chlorinating agent in the presence of a free-radical initiator and a solvent at from 0-120°C, and the solvent used is chlorobenzene, 1,2-dichlorobenzene, 1,3-dichlorobenzene, 1,4-dichlorobenzene, 1,2,3-trichlorobenzene, 1,2,4-trichlorobenzene, 1,3,5-trichlorobenzene, 2-chlorotoluene, 3-chlorotoluene, 4-chlorotoluene or a mixture of the same.

2. The process as claimed in claim 1, wherein the benzaldehyde of the formula (2) is used, where R independently of each other is identical or different and is halogen, NO₂, CN, NR'₂, OR', SO₂R' , SO₂OR', COR' or CO₂R', where R' is an unbranched or branched C₁-C₄ alkyl radical or an unsubstituted phenyl radical.

3. The process as claimed in claim 1, wherein a benzaldehyde (2) is used where x is 1.

4. The process as claimed in one or more of claims 1 to 3, wherein a benzaldehyde (2) is used where R is F, Cl or NO₂, x is 1 and at least one of the radicals R is an F or Cl in ortho position to the aldehyde group.

5. The process as claimed in one or more of claims 1 to 4, wherein the chlorinating agent used is Cl₂, SOCl₂, SO₂Cl₂, PCl₃, POCl₃, PCl₅, SbCl₅, ICl, ICl₃, SCl₂, S₂Cl₂, MnCl₄, (C₁-C₄) alkyl hypochlorite, CCl₄, N-chlorosuccinimide or a mixture of the same.

6. The process as claimed in one or more of claims 1 to 5, wherein the chlorinating agent used is Cl₂, SOCl₂, SO₂Cl₂ or a mixture of the same.

7. The process as claimed in one or more of claims 1 to 6, wherein the free-radical initiator used is an organic peroxide or an organic azo compound or a mixture of the same.

8. The process as claimed in one or more of claims 1 to 7, wherein 2,2'-azobis(2,4-dimethylvaleronitrile), 2,2'-azobis(isobutyronitrile), 2,2'-azobis(4-methoxy-2,4-dimethylvaleronitrile) or a mixture of the same is used as free-radical initiator.

9. The process as claimed in one or more of claims 1 to 8, wherein the reaction is carried out at from 20 to 90°C.

## Revendications

1. Procédé de fabrication de chlorures de benzoyle substitués de formule (1) 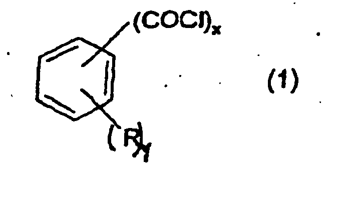 dans laquelle R, x et y ont la signification indiquée ci-dessous, **caractérisé en ce qu'**on utilise un benzaldéhyde de formule (2), dans lequel au moins un des radicaux R représente un halogène situé en position ortho par rapport à un groupe aldéhyde 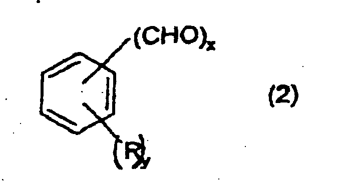 dans lequel R, indépendamment l'un de l'autre, est identique ou différent et représente un radical phényle non substitué ou un radical phényle substitué par un groupe -halogène, -NO₂, -CN, -alkyle en (C₁ à C₄) ou - alcoxy en (C₁ en C₄), un groupe -halogène, -NO₂, -CN, - NR'₂, -OR', -SO₂R', -SO₂OR', -COR' ou -CO₂R', R' étant un radical alkyle en C₁ à C₁₀ à chaîne droite ou ramifié, un radical phényle non substitué ou un radical phényle substitué par un groupe -halogène, -NO₂, -CN, -alkyle en (C₁ à C₄) ou -alcoxy en (C₁ à C₄), x représente 1 ou 2 et y 1, 2 ou 3, avec un agent de chloration en présence d'un initiateur radicalaire et d'un solvant à une température de 0-120°C et
**en ce qu'**on utilise, en tant que solvant, du chlorobenzène, du 1,2-dichlorobenzène, du 1,3-dichlorobenzène, du 1,4-dichlorobenzène, du 1,2,3-trichlorobenzène, du 1,2,4-trichlorobenzène, du 1,3,5-trichlorobenzène, du 2-chlorotoluène, du 3-chlorotoluène, du 4-chlorotoluène ou un mélange de ceux-ci.

2. Procédé selon la revendication 1, **caractérisé en ce qu'**on utilise un benzaldéhyde de formule (2) dans laquelle R, indépendamment l'un de l'autre, est identique ou différent et représente un groupe -halogène, -NO₂, - CN, -NR'₂, -OR', -SO₂R', -SO₂OR', -COR' ou -CO₂R', R' étant un radical alkyle en C₁-C₄ à chaîne droite ou ramifié ou un radical phényle non substitué.

3. Procédé selon la revendication 1, **caractérisé en ce qu'**on utilise un benzaldéhyde (2), dans lequel x représente 1.

4. Procédé selon l'une quelconque ou plusieurs des revendications 1 à 3, **caractérisé en ce qu'**on utilise un benzaldéhyde (2) dans lequel R représente -F, -Cl ou - NO₂, x représente 1 et au moins l'un des radicaux R représente un -F ou Cl situé en position ortho par rapport au groupe aldéhyde.

5. Procédé selon l'une quelconque ou plusieurs des revendications 1 à 4, **caractérisé en ce qu'**on utilise, comme agent de chloration du Cl₂, du SOCl₂, du SO₂Cl₂, du PCl₃, du POCl₃, du PCl₅, du SbCl₅, de l'ICl, de l'ICl₃, du SCl₂, du S₂Cl₂, du MnCl₄, de l'hypochlorite d'alkyle en (C₁ à C₄) , du CCl₄, du N-chlorosuccinimide ou un mélange de ceux-ci.

6. Procédé selon l'une quelconque ou plusieurs des revendications 1 à 5, **caractérisé en ce qu'**on utilise, comme agent de chloration du Cl₂, du SOC12, du SO₂Cl₂ ou un mélange de ceux-ci.

7. Procédé selon l'une quelconque ou plusieurs des revendications 1 à 6, **caractérisé en ce qu'**on utilise comme initiateur radicalaire un peroxyde organique ou un composé azo organique ou un mélange de ceux-ci.

8. Procédé selon l'une quelconque ou plusieurs des revendications 1 à 7, **caractérisé en ce qu'**on utilise du 2,2'-azobis(2,4-diméthylvaléronitrile), du 2,2'-azobis-(isobutyronitrile), du 2,2'-azobis(4-méthoxy-2,4-diméthylvaléronitrile) ou un mélange de ceux-ci en tant qu'initiateur radicalaire.

9. Procédé selon l'une quelconque ou plusieurs des revendications 1 à 8, **caractérisé en ce qu'**on réalise la conversion à une température de 20 à 90°C.
